(19) **Europäisches Patentamt**

**European Patent Office**

**Office européen des brevets**

(11) **EP 0 811 056 B1**

(12) **EUROPEAN PATENT SPECIFICATION**

(45) Date of publication and mention
of the grant of the patent:
**31.05.2000 Bulletin 2000/22**

(51) Int Cl.[7]: **C12N 5/02**

(86) International application number:
**PCT/EP96/00720**

(21) Application number: **96904839.6**

(22) Date of filing: **20.02.1996**

(87) International publication number:
**WO 96/26266 (29.08.1996 Gazette 1996/39)**

(54) **PEPTIDES FOR TISSUE AND CELL CULTURE MEDIA**

PEPTIDE FÜR GEWEBE- UND ZELLKULTURMEDIEN

PEPTIDES DESTINES A DES MILIEUX DE CULTURE TISSULAIRE ET CELLULAIRE

(84) Designated Contracting States:
**DE FR GB IT NL**

(30) Priority: **23.02.1995 US 393338**
**23.02.1995 US 393336**

(43) Date of publication of application:
**10.12.1997 Bulletin 1997/50**

(73) Proprietor: **QUEST INTERNATIONAL B.V.**
**1411 GP Naarden (NL)**

(72) Inventors:
• **BLOM, Wim, R.**
  **NL-3994 WJ Houten (NL)**
• **KUNST, Anthonie**
  **NL-1273 AN Huizen (NL)**
• **HAKKAART, Marcellinus, Jacobus, J.**
  **NL-1213 VN Hilversum (NL)**
• **LULI, Gregory, W.**
  **Schaumburg, IL 601955 (US)**
• **VAN SCHIE, Bartholomeus, Josef**
  **NL-1273 CP Huizen (NL)**

(74) Representative: **Kraag, F., Ir. et al**
**Nederlandsch Octrooibureau**
**Scheveningseweg 82**
**P.O. Box 29720**
**2502 LS Den Haag (NL)**

(56) References cited:
**EP-A- 0 220 379**          **US-A- 4 235 772**

• **Database PAJ; 8 May 1990 MIHARA AKIRA ET AL.: "Culture medium" & JP-A-2049579 (Kyowa Hakko Kogyo Co. Ltd.) 19 February 1990 XP002006666**
• **JOURNAL OF IMMUNOLOGICAL METHODS, vol. 166, no. 1, 1993, NEW YORK US, pages 85-91, XP002006664 S. HEENEMAN ET AL.: "The concentrations of glutamine and ammonia in comercially available cell culture media" cited in the application**
• **JOURNAL OF FOOD BIOCHEMISTRY, vol. 16, no. 4, 1993, pages 235-248, XP002006665 SOICHI TANABE ET AL.: "Production of a high-glutamine oligopeptide fraction from gluten by enzymatic treatment and evaluation of its nutritional effect on the small intestine of rats " cited in the application**

**Description**

Background of the invention

The present invention relates to a novel method for proliferating, propagating, maintaining and culturing of eucaryotic cells and to suitable culture media for this purpose. In particular the present invention relates to such method comprising the use of protein hydrolysate, prepared from a protein using one or more hydrolytic enzymes, as a basis of a medium for eucaryotic cells and to culture media comprising such protein hydrolysate.

Description of related art

Existing media for in vitro culturing of eucaryotic cells (culture media) in general comprise mixtures of amino acids, vitamins, carbohydrates and minerals. The prior art has described the need for the amino acid L-glutamine as an essential ingredient in such media. Illustrative of this art is US 3,579,423 (Yamane et al). Culture media contain relatively large amounts of L-glutamine. Typically it is used in a cell growth and maintenance medium at a concentration of about 2 mM. It is an important energy source in proliferating eucaryotic cells and it also serves as both a carbon and a nitrogen source, especially for purine and pyrimidine synthesis.

The use of glutamine as an energy source in cultured mammalian cells proceeds via deamidation of glutamine by glutaminase to yield glutamate and ammonia. Glutamate then undergoes transamination to produce α-ketoglutaric acid which is incorporated into the energy yielding Krebbs tricarboxylic acid cycle.

The incorporation of glutamine in a liquid cell culture medium however suffers from the disadvantage that glutamine is not very stable in the free amino acid form. It is well known to rapidly decompose into ammonia and pyroglutamic acid. Recently Heeneman et al (J. Immunological Methods 166, 85-91, 1993) found that as a consequence of this decomposition all tested commercial media contained significantly less glutamine than prescribed. In addition Heeneman et al point to the fact that the formed ammonia can be toxic to cultured cells.

When glutamine is incorporated in a peptide it does not decompose, provided that the glutamine residue is not present at the amino terminal end since only in this position the glutamine residue can decompose into a pyroglutamic acid residue and ammonia.

The prior art has described the use of small synthetic peptides for cell growth in US 4,235,772 (Hugo) and US 4,058,512 (Sievertsson et al). These peptides contain small amounts of L-glutamine and are thus not a good source for this essential amino acid. Heeneman (vide supra) and K. Brand et al (Metabolism 38(8), Suppl.1, 1989 29-33) recommend the use of the dipeptide Glycyl-L-glutamine as a source of glutamine. Brand, however, reports that on a glutamine basis more of the dipeptide (up to 6 times) is needed than of free glutamine to obtain the same result. This dipeptide can only be suitably obtained by synthesis. All synthetic peptides suffer from the disadvantage that they are expensive and have limited availability.

There is abundant prior art on the preparation of peptides by hydrolysis of protein. In general two types of such protein hydrolysates can be distinguished: (1) hydrolysates comprising peptides with a chain length above 15 amino acids and a relatively low level of free amino acids (generally below 10%); and (2) hydrolysates comprising peptides with a chain length below 15 amino acids and a relatively high level of free amino acids (about 20% or more).

Hydrolysates of the first group are used in food applications as functional ingredients e.g. emulsifiers or aeration aids. It is well known that for optimal properties peptide chains above 15 amino acid residues (e.g. 15-50) are required. The presence of free amino acids should be avoided as these give an unwanted savoury taste and smell to the product. Consequently these type of hydrolysates comprise peptides with a chain length well above 15 amino acids and a level of free amino acids below about 10%.

Hydrolysates of the second group are in food applications mainly used in infant and clinical nutrition formulae where a low allergenicity and preferably reduced bitterness is required. For these purposes the product should contain small peptides and this is achieved with enzyme preparations having both endo- and exo-peptidase activity. Due to the action of the exopeptidases the amount of free amino acids is strongly increased to levels of about 20% or higher.

In the fermentation industry hydrolysates of the second group with high amounts of free amino acids (20% or higher) are used as a relatively cheap source of amino acids in culture media for microorganisms. Protein hydrolysates with a high level of free amino acids, however, also suffer from the disadvantage that free glutamine decomposes into pyroglutamic acid and the toxic ammonia and thus, they are not very well suited for application in eucaryotic cell culture media.

The prior art (e.g. Animal Cell Culture, A practical approach, second edition, ISBN 0-19-963213-8) describes the use of lactalbumin hydrolysates (prepared with pancreatin which contains both endo- and exopeptidase activity) or peptones (hydrolysates with a very high level of free amino acids) in cell culture media, but only as supplements and not as the main source of glutamine or other amino acids.

The prior art has described various hydrolysates derived from wheat gluten, which contain the glutamine residue in the molecule; illustrative are US 3,852,479 (Yokotsuka et al), 3,932,671 (Yokotsuka et al) and 4,100,151 (Adler-Nissen) and JP 02-049579 (Mihara et al). These products are used in foods. Tanabe et al, J. Food Biochem., 16(4), 1993 235-48 investigated the use of a high-glutamine oligopeptide, obtained by hydrolysis of gluten, as a glutamine source in enteral nutrition through rat feeding studies..

There appears to be no description in the prior art of the use in culture media for eucaryotic cells of higher peptides than (synthetic) dipeptides, or of intact proteins containing glutamine as the main or only source of glutamine and/or other amino acids.

Objects of the invention

It is an object of the present invention to provide a novel method for in vitro culturing of eucaryotic cells. In particular it is an object of the present invention to economically provide amino acids, particularly glutamine, in a form in which they can be taken up by eucaryotic cells which is stable in aqueous solution under the conditions used to culture eucaryotic cells, which is easy to use, can be sterilised by conventional means and is free of toxic and inhibitory effects. Furthermore, it is an object of the present invention to provide stable, glutamine providing culture media for culturing eucaryotic cells, which are easy and economic to produce. Other objects of the invention will become apparent to those skilled in the art.

Detailed description of the invention

The present invention provides a method for in vitro maintaining or growing eucaryotic cells by use of a culture medium comprising a glutamine containing protein hydrolysate, obtained by enzymatic hydrolysis of a protein material, wherein the protein hydrolysate has a free amino acid level of less than 15% by weight of the total proteinaceous material, an average chain length of the peptides of less than 15 amino acids and wherein at least 90% by weight of the protein hydrolysate has a molecular weight below 1000 D as determined by gel permeation chromatography.

Furthermore, the invention provides culture media for in vitro maintaining or growing eucaryotic cells which comprise a glutamine containing protein hydrolysate, obtained by enzymatic hydrolysis of a protein material, wherein the protein hydrolysate has a free amino acid level of less than 15% by weight of the total proteinaceous material, an average chain length of the peptides of less than 15 amino acids and wherein at least 90% by weight of the protein hydrolysate has a molecular weight below 1000 D as determined by gel permeation chromatography.

The glutamine containing protein hydrolysates used for the purposes of this invention are obtained according to methods known in the art i.e. by enzymatic hydrolysis of protein, which may be of plant or animal origin, such as milk protein (casein, albumin, etc.), meat protein, soy protein or cereal protein (wheat, rice, maize, etc.). Since most commonly used cell culture media contain high levels of glutamine, the protein hydrolysate for the purposes of this invention should preferably have a level of glutamine residues of 20% by weight or more. A protein source which is high in glutamine residues is therefore generally preferred as the starting material, such as cereal protein, more particularly wheat gluten or its subfractions glutenin and gliadin which are known to contain 25-30% of glutamine.

The hydrolytic enzyme or enzymes used for the hydrolysis of the protein starting material may be of animal, plant, yeast, bacterial or fungal origin. Preferably enzymes are used which have a low exo-peptidase activity so as to minimize the liberation of free amino acids. Suitable enzymes are e.g. Pepsin, Alcalase or Orientase.

The protein hydrolysates should have a level of free amino acids below 15% by weight, preferably below 10%. For specific purposes a level of 4% or less may be required. At least 90% by weight of the protein hydrolysate has a molecular weight below 1000 D. Also, the average peptide chain length in the protein hydrolysate is below 15 amino acid residues, and preferably below 10. On the other hand the minimum molecular weight of the peptides should preferably be above 200 D.

Conventional culture media contain vitamins, a carbohydrate source and (a source of) amino acids. The pH of the media preferably is between 6 and 8. As outlined above, the prior art has used various hydrolysed proteins such as milk protein (casein) for culture media intended for culturing microorganisms. Microorganisms have the necessary enzymes to enable growth on complex protein. However, higher eucaryotic cells, though comprising subsets of cells having vastly different characteristics, in general lack the capacity for utilising complex protein. It was thus unexpected that the protein hydrolysates according to the present invention were able to support the culturing of eucaryotic cells. There is no clear explanation for this observation, but it may be hypothesised that eucaryotic cells apart from the sodium dependant amino acid uptake system (comprising a carrier protein) possess a mechanism via which small peptides can be transported into the cell.

The culture media of the present invention include ingredients conventionally found in media for culturing eucaryotic cells i.e. vitamins, minerals, carbohydrates, growth promotors and amino acids. Rather than free L-glutamine they contain the protein hydrolysates as the main or only source thereof. The protein hydrolysates can also be used as the source of other essential amino acids in the culture media.

The eucaryotic cells are preferably animal cells, more preferably mammalian (such as human) or insect cells.

Eucaryotic cells are often cultured with the aim of having them produce certain valuable compounds, particularly for pharmaceutical or diagnostic purposes. It is also well known to those skilled in the art that eucaryotic cell cultures generally show a growth phase, in which the number of cells increases, followed by a stationary phase in which the number of cells remains more or less constant. It is often in this stationary phase that production of the desired compounds is greatest in the culture. It has been found that the protein hydrolysates according to the invention not only act favourably on the growth phase, but particularly also on the production phase, i.e. increase the production of the desired compound by a given cell mass in a given time, compared with prior art culture media containing free glutamine.

The culture media according to the present invention may be provided as complete kits including a container in which the cells to be cultured can be introduced. The culture medium can be supplied as a dry mix to which water is added to produce a liquid culture medium ready for use. Alternatively the medium may be provided as a ready to use sterile liquid to which the cells may be added directly. The latter obviates the need for sterilization of the medium after preparation. Unlike the prior art media the liquid media according to the invention may be shipped and stored without deterioration, due to the stable form in which glutamine is present.

The analytical methods to determine the various relevant parameters are described below.

**Analytical methods.**

Definitions

TN : Total Nitrogen.
AN : Alpha amino Nitrogen.
EN : Epsilon amino Nitrogen.
AEN : The sum of alpha and epsilon nitrogen.
PN : Nitrogen in (potential) peptide bonds (PN thus includes all AN).
FAA : free amino acid level.
F : average amount of Nitrogen per amino acid residue in a protein.
PCL : average peptide chain length.

Determination of parameters

AEN can be determined via methods such as the TNBS method (cf. J. Adler-Nissen, Enzymatic Hydrolysis of Food Proteins, Elsevier Applied Science Publishers, 1986) or via formol titration.
TN can be determined via the well known Kjeldahl method.
EN is only present in the side chain of lysine so it is equal to the amount of lysine in the product.
FAA is determined using an amino acid analyser.
AN can be calculated from the AEN (as determined via TNBS or formol titration) and the amount of lysine (=EN) in the protein hydrolysate:

$$AN = AEN - EN \tag{1}$$

PN can be approximated from TN using the average amount of nitrogen (F) per amino acid.

$$PN = TN/F \tag{2}$$

Most amino acids only have an alpha nitrogen atom but Trp, Lys, Asn and Gln have 1 extra nitrogen in the side chain, his has 2 extra nitrogen and arg has 3 extra nitrogen in the side chain. In Table 1 the average amounts of N per amino acid (F) for a number of common proteins are given.

Table 1.

| Some data on common proteins. | | |
|---|---|---|
| | F | % lysine |
| casein | 1.32 | 8.23 |
| Whey protein | 1.26 | 10.12 |
| Soy protein | 1.28 | 6.50 |
| Gluten | 1.43 | 1.58 |

Calculation of the average peptide chain length

The average peptide chain length can be calculated from AN and PN:

$$PCL = PN/AN \qquad (3)$$

Combining eq. 3 with eq. 2 gives:

$$PCL = TN/(F*AN) \qquad (4)$$

Combining eq. 4 with eq. 1 gives:

$$PCL = TN/(F * (AEN-EN)) \qquad (5)$$

With eq. 5 the average peptide length in a hydrolysate is calculated in which also the FAA is taken into account. Strictly spoken an amino acid is not a peptide and FAA thus should not be included in the calculation of the average PCL. To calculate the average peptide length of the non FAA fraction, TN and AN of this fraction are required. Rewriting eq. 4 for the peptide fraction gives:

$$PCL_{pep} = TN_{pep}/(F*AN_{pep}) \qquad (6)$$

in which:

$$TN_{pep} = TN - TNFAA \qquad (7)$$

$$TN_{FAA} = F * FAA \qquad (8)$$

$$AN_{pep} = AN - FAA \qquad (9)$$

Combining eq. 6 with eq 1, 7, 8 and 9 results in:

$$PCL_{pep} = \frac{TN/F - FAA}{AEN - EN - FAA} \qquad (10)$$

in which TN, AN, AEN, EN and FAA are given in mmol per weight unit.

**2. Determination of molecular weight distribution.**

There are a number of methods to determine the molecular weight distribution. An easy and convenient method uses gel permeation chromatography. There are many, all slightly different procedures reported in the literature. For the purposes of this invention use was made of a Protein-Pak 60 (Trade Mark) column from Waters with a length of 30 cm and an internal diameter of 7.8 mm and a Protein-Pak 125 (Trade Mark) Bulk Packing guard column. The column is eluted with a 0.1 M potassium phosphate buffer with pH 7.0 at a flow rate of 1.0 ml/min. For analysis 20 μl samples containing 0.2-0.5 mg product per ml elution buffer are injected on the column. Protein and peptide peaks are detected at 214 nm. The amount of material within a molecular weight range is determined from the surface under the chromatogram in that molecular weight range.

**3. Determination of glutamine levels in protein hydrolysates**

Due to the instability of free glutamine it is not possible to determine the amount of glutamine in a protein based product via the normal procedure to determine the amino acid composition. In this procedure the protein based product is treated with 6N HCl to hydrolyse it into free amino acids of which the amount can then be determined with an amino acid analyser. During the 6N HCl hydrolysis glutamine decomposes into ammonia and pyroglutamate which is subsequently converted in glutamic acid.

An indirect method to analyse the amount of glutamine in a hydrolysate is to determine the amount of $NH_3$ liberated during the acid hydrolysis as described by MacRitchie (J. Food Technol. **14**, 595-601, 1979). Since $NH_3$ is not only liberated from glutamine but also from asparagine (which decomposes into ammonia and aspartic acid) the amount of mmol $NH_3$ liberated from a protein sample equals the amount of mmol asparagine + glutamine (Asn+Gln) in that protein sample. Since the origin of the liberated $NH_3$ cannot be determined it has to be assumed that the proportion of amidated groups is the same in the two types of chains.

We have tested the reliability of this method by determination of the amount of $NH_3$ liberated from casein and whey protein and comparing the results with the theoretical results calculated on basis of the known compositions and amino acid sequences of the individual caseins and whey proteins. In addition the amount of $NH_3$ liberated from gluten was determined. The results are summarised in Table 2. It can be seen that there is a good agreement between the (Asn+Gln)/(Asx+Glx) ratio as determined experimentally from the analysed amounts of liberated $NH_3$, Glx (glutamine + glutamic acid) and Asx (asparagine + aspartic acid) and the ratio as it should theoretically be on basis of the amino acid composition. The experimentally determined ratio for gluten (77.3%) is in line with the ratio reported by MacRitchie (75.8%).

Table 2.

| Determination of Gln levels. | | | |
|---|---|---|---|
| | Casein | Whey | Gluten |
| %TN | 13.7 | 14.4 | 14.0 |
| mmol $NH_3$ (=mmol Asn+Gln) | 100.0 | 73.8 | 211.8 |
| mmol Asx (analytical data) | 49.9 | 80.2 | 18.2 |
| mmol Glx (analytical data) | 140.7 | 128.1 | 239.1 |
| % (Asn+Gln)/(Asx+Glx) (analytical data) | 52.5 | 35.4 | 77.3 |
| % (Asn+Gln)/(Asx+Glx) (Amino acid sequence) | 50.1 | 38.3 | |

From these results it is concluded that the determination of the amount of $NH_3$ liberated from a protein or a protein hydrolysate is a suitable method to assess the (Asn+Gln)/(Asx+Glx) ratio in a protein based product.

The following are non-limiting examples of the present invention.

EXAMPLE 1.

Production of a protein hydrolysate using vital gluten.

An 8% dispersion of vital gluten is hydrolysed with 1% (E/S) the commercially available enzyme preparation Orientase 90N (Trade Mark) (neutral protease ex Quest International Cork, Ireland) at 50°C for 4 hours. The pH is initially set at 7 and during hydrolysis it is not controlled. After hydrolysis the enzyme is inactivated via a heat treatment at 95°C for 1 minute. Residual intact protein and insoluble components are removed via centrifugation for 5 min. at 2500G and the obtained effluent is subsequently ultrafiltered. Preferably membranes with a mol. weight cutoff of 10.000 Dalton are used. The obtained ultrafiltration permeate is concentrated via evaporation and is then spray dried or freeze dried.

The obtained final product is characterised using the above described methods. The results are summarised in Table 3.

Table 3.

| % AN | 1.57 | |
|---|---|---|
| % TN | 14.80 | |
| % FAA | 3.54 | |
| PCL | 9.3 | |
| > 10 kD | 0.1 % | |
| 5-10 kD | 0.1 % | |
| 1-5 kD | 2.4 % | |
| <1 kD | 97.4% | |
| | Total Amino Acids - mg/g | Free Amino Acids - mg/g |
| Alanine | 23.4 | 1.9 |
| Arginine | 27.0 | 3.7 |
| Asparagine + Aspartic acid | 24.5 | 0.8 |
| Cysteine | 9.0 | 2.1 |
| Glutamine + Glutamic acid | 279.7 | 3.2 |
| Glycine | 29.9 | 0.3 |
| Histidine | 17.9 | 1.0 |
| Isoleucine | 24.7 | 1.9 |
| Leucine | 61.3 | 6.1 |
| Lysine | 12.1 | 2.0 |
| Methionine | 12.1 | 1.1 |
| Phenylalanine | 43.4 | 3.1 |
| Proline | 124.8 | 2.1 |
| Serine | 50.6 | 1.2 |
| Threonine | 24.0 | 0.8 |
| Tyrosine | 28.5 | 0.4 |
| Valine | 28.4 | 3.7 |

Evaluation of the hydrolysate in cell tissue cultures.

Three media were composed on basis of the well known RPMI-1640 medium. This medium was prepared as prescribed from the RPMI-1640 select Amine kit from Gibco BRL, Life Technologies Inc., Cat No. 17402-017. The medium was divided in three equal portions which were used as basis for medium 1, 2 and 3.

To the media the My additive can be added. This additive contains:

| L-glutamine | 2 mM |
|---|---|
| Sodium pyruvate | 1 mM |
| Gentamycin | 55 µg/ml |
| β-mercapto-ethanol | 50 µM |

(continued)

| | |
|---|---|
| hypoxanthine | 100 μM |
| thymidine | 15 μM |
| fetal bovine serum | 8% (v/v) |

Medium 1.

To the basic RPMI 1640 medium obtained from the Gibco's select Amine kit the My additive was added.

Medium 2.

To the basic RPMI-1640 medium obtained from the Gibco's select Amine kit the My additive without the L-glutamine was added. In stead of L-glutamine as present in the My additive 2.2 g/l of the obtained gluten hydrolysate was added.

Medium 3.

To the basic RPMI-1640 medium obtained from the Gibco's select Amine kit the My additive without the L-glutamine was added. Instead thereof 2.2 g/l of a mixture of free amino acids with the same composition as the obtained gluten hydrolysate was added.

The three media were used to culture following cell lines:

- U266 : a human myeloma cell line
- SP2/0 : a mouse myeloma cell line
- Anti CD20 : a hybridoma cell line.

To 0.5 ml of a cell suspension which was grown on a standard medium 4.5 ml of medium 1, 2 or 3 was added. After two days 5 ml fresh medium 1, 2 or 3 was added.
Cell counts were determined directly after adding medium, after 1, 5 and 7 days. The results are summarised in Table 4.
From the results it can be seen that the gluten hydrolysate from this invention does not give an acute cytotoxicity and that the cells can be cultured using the gluten hydrolysate according to this invention.

Table 4.

| Cell counts | | | | | |
|---|---|---|---|---|---|
| Counts are given in cells per ml. | | | | | |
| Cell line | Medium | day 0 | after 1 day | after 5 days | after 7 days |
| U266 | medium 1 | $1.7 * 10^5$ | $1.5 * 10^5$ | $1.6 * 10^5$ | $2.6 * 10^5$ |
| U266 | medium 2 | $1.2 * 10^5$ | $1.2 * 10^5$ | $1.9 * 10^5$ | $2.0 * 10^5$ |
| U266 | medium 3 | $1.1 * 10^5$ | $1.3 * 10^5$ | $1.4 * 10^5$ | $3.1 * 10^5$ |
| SP2/0 | medium 1 | $1.6 * 10^5$ | $1.4 * 10^5$ | $12.1 * 10^5$ | $16.7 * 10^5$ |
| SP2/0 | medium 2 | $0.7 * 10^5$ | $1.5 * 10^5$ | $12.5 * 10^5$ | $20.0 * 10^5$ |
| Sp2/0 | medium 3 | $0.8 * 10^5$ | $0.9 * 10^5$ | $14.5 * 10^5$ | $19.8 * 10^5$ |
| Anti CD20 | medium 1 | $0.8 * 10^5$ | $1.3 * 10^5$ | $12.9 * 10^5$ | $1.5 * 10^5$ |
| Anti CD20 | medium 2 | $1.0 * 10^5$ | $1.2 * 10^5$ | $11.7 * 10^5$ | $16.5 * 10^5$ |
| Anti CD20 | medium 3 | $0.9 * 10^5$ | $1.7 * 10^5$ | $11.7 * 10^5$ | $13.5 * 10^5$ |

EXAMPLE 2.

Production of a protein hydrolysate using vital gluten.

An 8% dispersion of vital gluten is hydrolysed with 0.1% (E/S) of the commercially available enzyme preparation pepsin orthana 1:10,000 NF (PCA Diagnostica, Haarlem, The Netherlands) at 50°C for 16 hours. The pH is initially set at 1.5 with hydrochloric acid and is not controlled during further hydrolysis. After hydrolysis the enzyme is inactivated via a heat treatment at 95°C for 1 minute.

Residual intact protein and insoluble components are removed via centrifugation and the obtained effluent is subsequently ultrafiltered. Preferably membranes with a mol. weight cutoff of 10.000 Dalton are used. The obtained ultrafiltration permeate is concentrated via evaporation and is then spray dried or freeze dried.

The obtained final product is characterised using the above described methods. The results are summarised in Table 5.

Table 5.

| % AN | 0.90 | |
|---|---|---|
| % TN | 12.30 | |
| % FAA | 0.70 | |
| PCL | 11.9 | |
| > 10 kD | 1.1 | |
| 5-10 kD | 1.0 | |
| 1-5 kD | 7.4 | |
| <1 kD | 90.5 | |
| | Total amino acids mg/g | Free amino acids - mg/g |
| Alanine | 25.4 | 0.7 |
| Arginine | 24.8 | 0.0 |
| Asparagine + aspartic acid | 33.9 | 0.8 |
| Cysteine | 4.4 | 2.1 |
| Glutamine + glutamic acid | 162.9 | 0.0 |
| Glycine | 21.3 | 0.1 |
| Histidine | 13.5 | 0.0 |
| Isoleucine | 25.6 | 0.0 |
| Leucine | 60.8 | 0.5 |
| Lysine | 15.2 | 0.1 |
| Methionine | 13.3 | 0.1 |
| Phenylalanine | 32.7 | 0.7 |
| Proline | 55.0 | 1.1 |
| Serine | 39.2 | 0.2 |
| Threonine | 23.0 | 0.1 |
| Tryptophan | 24.3 | 0.0 |
| Tyrosine | 28.6 | 0.4 |
| Valine | 34.1 | 0.1 |

Evaluation of the hydrolysate in cell tissue cultures.

Two media were composed on basis of the well known RPMI-1640 medium. This medium was prepared from the RPMI-1640 select Amine kit from Gibco BRL, Life Technologies Inc., Cat No. 17402-017. The medium was divided in two equal portions which were used as basis for medium 1 and 4.

Medium 1.

To the basic RPMI-1640 medium obtained from the Gibco's select Amine kit the My additive was added as in example 1.

Medium 4.

To the basic RPMI-1640 medium obtained from the Gibco's select Amine kit the My additive without the L-glutamine was added. In stead of L-glutamine as present in the My additive 3.1 g/l of the obtained gluten hydrolysate was added.

The two media were used to culture the cell lines described in example 1 following the procedure described in example 1. The results are summarised in Table 6.

Table 6.

| Cell counts | | | | | |
|---|---|---|---|---|---|
| Counts are given in cells per ml. | | | | | |
| Cell line | Medium | day 0 | after 1 day | after 5 days | after 7 days |
| U266 | medium 1 | $1.7 * 10^5$ | $1.5 * 10^5$ | $1.6 * 10^5$ | $2.6 * 10^5$ |
| U266 | medium 4 | $1.4 * 10^5$ | $1.1 * 10^5$ | $1.3 * 10^5$ | $1.3 * 10^5$ |
| SP2/0 | medium 1 | $1.6 * 10^5$ | $1.4 * 10^5$ | $12.1 * 10^5$ | $16.7 * 10^5$ |
| SP2/0 | medium 4 | $1.6 * 10^5$ | $1.5 * 10^5$ | $13.6 * 10^5$ | $14.9 * 10^5$ |
| Anti CD20 | medium 1 | $0.8 * 10^5$ | $1.3 * 10^5$ | $12.9 * 10^5$ | $1.5 * 10^5$ |
| Anti CD20 | medium 4 | $1.1 * 10^5$ | $1.2 * 10^5$ | $14.0 * 10^5$ | $11.4 * 10^5$ |

From the results it can be seen that the gluten hydrolysate from this invention does not give an acute cytotoxicity and that the cells can be cultured using the gluten hydrolysate of this invention.

EXAMPLE 3

Influence of the hydrolysate on production in cell tissue cultures.

The protein hydrolysate described in example 1 was used to compose three media. These media were prepared from the RPMI-1640 select Amine kit from Gibco BRL, Life Technologies Inc., Cat. No. 17402-017 (Glasgow, Scotland).

To the media a supplement was added containing:

| | |
|---|---|
| Sodium pyruvate | 1 mM |
| Gentamycin | 55 µg/ml |
| β-Mercaptoethanol | 50 µM |
| Hypoxanthine | 100 µM |
| Thymidine | 15 µM |
| Fetal bovine serum | 8 (v/v) |

Medium 1

The basic RPMI-1640 medium. This medium contains 2 mM glutamine

Medium 2

To the basic RPMI-1640 medium, without free amino acids, 1.07 g/l of the gluten hydrolysate described in Example 1, is added. Thus, the medium contains about 2 mM glutamine residues. Additionally the free amino acids asparagine, aspartic acid, glutamic acid, cystine, methionine, isoleucine, lysine, arginine and hydroxyproline were supplemented in small amounts to compensate for the low levels of these amino acids in the obtained gluten hydrolysate.

Medium 3

To the basic RPMI-1640 medium, without free amino acids, 2.14 g/l of the gluten hydrolysate described in Example 1, is added. Additionally the free amino acids asparagine, aspartic acid, glutamic acid, cystine, methionine, isoleucine, lysine, arginine and hydroxyproline were supplemented in small amounts to compensate for the low levels of these amino acids in the obtained gluten hydrolysate.

Medium 4

To the basic RPMI-1640 medium, without free amino acids, 4.28 g/l of the gluten hydrolysate described in Example 1, is added. Additionally the free amino acids asparagine, aspartic acid, glutamic acid, cystine, methionine, isoleucine, lysine, arginine and hydroxyproline were supplemented in small amounts to compensate for the low levels of these amino acids in the obtained gluten hydrolysate.

The above media were used to study the growth performance of, as well as production by the hybridoma cell line Anti CD-20. This cell line was chosen because it is an antibody producing cell line with high nutritional requirements. The cells were grown on the formulated media in triplo on 25 cm$^2$ flasks. The media were refreshed at day 7. The results are summarized in Table 7

Table 7

| Cell counts (expressed as cells per ml). | | | | |
|---|---|---|---|---|
| Day | Medium 1 | Medium 2 | Medium 3 | Medium 4 |
| day 0 | $0.67*10^5$ | $0.67*10^5$ | $0.67*10^5$ | $0.67*10^5$ |
| after 1 day | $1.0*10^5$ | $0.8*10^5$ | $1.0*10^5$ | $0.9*10^5$ |
| after 2 days | $1.68*10^5$ | $1.58*10^5$ | $1.26*10^5$ | $1.26*10^5$ |
| after 3 days | $5.66*10^5$ | $3.44*10^5$ | $4.44*10^5$ | $4.55*10^5$ |
| after 6 days | $3.17*10^5$ | $4.82*10^5$ | $7.05*10^5$ | $10.4*10^5$ |
| after 6 days | $0.48*10^5$ | $0.72*10^5$ | $1.1*10^5$ | $1.6*10^5$ |
| after 7 days | $1.0*10^5$ | $0.9*10^5$ | $3.0*10^5$ | $5.9*10^5$ |
| after 8 days | $3.79*10^5$ | $2.63*10^5$ | $5.89*10^5$ | $10.4*10^5$ |
| after 9 days | $7.44*10^5$ | $5*10^5$ | $6.66*10^5$ | $13.3*10^5$ |
| after 10 days | $11.76*10^5$ | $4.35*10^5$ | $8.23*10^5$ | $14.1*10^5$ |
| after 14 days | $0.6*10^5$ | $3.12*10^5$ | $2.5*10^5$ | $4.12*10^5$ |

All the test conditions of table 7 were analysed with ELISA on the antibody production. The results are summarized in Table 8

Table 8

| Antibody production of hybridoma Anti CD-20 on the formulated media. The antibody concentrations IgG of the supernatants are expressed in µg/ml. | | | | |
|---|---|---|---|---|
| Day | Medium 1 | Medium 2 | Medium 3 | Medium 4 |
| after 1 day | 0.9 | 1.9 | 1.8 | 1.6 |
| after 2 days | 2.2 | 1.9 | 3.0 | 3.3 |
| after 3 days | 5.0 | 2.8 | 5.7 | 4.0 |
| after 6 days | 18.9 | 9.0 | 30.6 | 22.4 |
| after 7 days | 5.2 | 2.9 | 10.7 | 8.1 |
| after 8 days | 8.5 | 5.2 | 18.7 | 13.4 |
| after 9 days | 9.7 | 5.2 | 22.2 | 23.3 |
| after 10 days | 13.7 | 6.8 | 21.6 | 16.0 |

Table 8 (continued)

| Day | Medium 1 | Medium 2 | Medium 3 | Medium 4 |
|---|---|---|---|---|
| after 14 days | 23.8 | 9.5 | 51.0 | 44.4 |

EXAMPLE 4

Influence of the hydrolysate in cell tissue cultures.

The protein hydrolysate described in Example 1 was used to compose two media. These media was prepared from the RPMI-1640 select Amine kit from Gibco BRL, Life Technologies Inc., Cat. No. 17402-017 (Glasgow, Scotland).
To the media a supplement was added. This supplement contains:

| | |
|---|---|
| Sodium pyruvate | 1 mM |
| Gentamycin | 55 µg/ml |
| β-Mercaptoethanol | 50 µM |
| Hypoxanthine | 100 µM |
| Thymidine | 15 µM |
| Fetal bovine serum | 8 % (v/v) |

Medium 1

The basic RPMI-1640 medium, which contains 2 mM glutamine.

Medium 2

To the basic RPMI-1640 medium, without free amino acids, 1.07 g/l of the gluten hydrolysate described in example 1, is added. Thus, the medium contains about 2 mM glutamine residues.

Medium 3

To the basic RPMI-1640 medium, without free amino acids, 2.14 g/l of the gluten hydrolysate described in example 1, is added. Thus, this medium contains about 4mM glutamine residues.
The above media were used to study the growth performance of, as well as production by the hybridoma cell line Anti CD-20. The cells were grown on the formulated media in triplo on 25 cm$^2$ flasks. The media were refreshed at day 7. The results are summarized in Table 9

Table 9.

| Cell counts | | | |
|---|---|---|---|
| Counts are given in cells per ml. | | | |
| Day | Medium 1 | Medium 2 | Medium 3 |
| day 0 | $1.3*10^5$ | $1.3*10^5$ | $1.3*10^5$ |
| after 1 day | $1.9*10^5$ | $1.8*10^5$ | $2.0*10^5$ |
| after 2 days | $5.05*10^5$ | $5.89*10^5$ | $5.7*10^5$ |
| after 3 days | $10.1*10^5$ | $6.88*10^5$ | $6.0*10^5$ |
| after 4 days | $14.1*10^5$ | $4.47*10^5$ | $5.1*10^5$ |
| after 9 days | $2.0*10^4$ | $1.0*10^4$ | $1.3*10^5$ |

All the test conditions of table 9 were analysed with ELISA on the antibody production. The results are summarized in Table 10.

Table 10

| Antibody production of Hybridoma Anti CD-20 on the formulated media. The antibody concentration IgG of the supernatants were expressed in µg/ml. | | | |
|---|---|---|---|
| Day | Medium 1 | Medium 2 | Medium 3 |
| after 1 day | 1.9 | 2.7 | 4.4 |
| after 2 days | 4.4 | 6.0 | 7.9 |
| after 3 days | 8.3 | 10.4 | 11.2 |
| after 4 days | 13.6 | 12.7 | 16.8 |
| after 9 days | 18.5 | 13.0 | 52.1 |

Example 3 as well as Example 4 show that using the protein hydrolysate as the source of glutamine in most cases gives improved growth compared to the standard RPMI-1640 culture medium containing free amino acids. The effect on production of antibodies is even more pronounced, even in those cases where the number of cells is not markedly different between the standard medium and the media containing the protein hydrolysate according to the invention.

**Claims**

1.  A method for in vitro maintaining or growing eucaryotic cells by use of a culture medium comprising a glutamine containing protein hydrolysate, obtained by enzymatic hydrolysis of a protein material, wherein the protein hydrolysate has:

    -    a free amino acid level of less than 15% by weight of the total proteinaceous material,
    -    an average chain length of the peptides of less than 15 amino acids,
    -    and wherein at least 90% by weight of the protein hydrolysate has molecular weight below 1000 D as determined by gel permeation chromatography.

2.  A method according to claim 1 wherein the average peptide chain length in the protein hydrolysate is less than 10 amino acid residues.

3.  A method according to claims 1-2 wherein the protein hydrolysate has a free amino acid level of less than 4% by weight of the total proteinaceous material.

4.  A method according to claims 1-3 wherein the protein hydrolysate contains 20% by weight or more of glutamine residues.

5.  A method according to claims 1-4 wherein the protein material is a cereal protein.

6.  A method according to claim 5 wherein the protein material is a wheat gluten or a subfraction thereof.

7.  A culture medium for in vitro maintaining or growing eucaryotic cells which comprises a glutamine containing protein hydrolysate, obtained by enzymatic hydrolysis of a protein material, wherein the protein hydrolysate has:

    -    a free amino acid level of less than 15% by weight of the total proteinaceous material,
    -    an average chain length of the peptides of less than 15 amino acids
    -    and wherein at least 90% by weight of the protein hydrolysate has molecular weight below 1000 D as determined by gel permeation chromatography.

8.  A culture medium according to claim 7 wherein the average peptide chain length in the protein hydrolysate is less than 10 amino acid residues.

9.  A culture medium according to claims 7-8 wherein the protein hydrolysate has a free amino acid level of less than 4% by weight of the total proteinaceous material.

10. A culture medium according to claims 7-9 wherein the protein hydrolysate contains 20% by weight or more of glutamine residues.

11. A culture medium according to claims 7-10 wherein the protein material is a cereal protein.

12. A culture medium according to claim 11 wherein the protein material is a wheat gluten or a subfraction thereof.

**Patentansprüche**

1. Verfahren für die in-vitro-Erhaltung oder das -Wachstum von eukaryontischen Zellen durch Verwendung eines Kulturmediums, umfassend ein Glutamin-enthaltendes Proteinhydrolysat, welches durch enzymatische Hydrolyse eines Proteinmaterials erhalten wird, wobei das Proteinhydrolysat

   - ein freies Aminosäurelevel von weniger als 15 Gew.-% des gesamten proteinhaltigen Materials,
   - eine durchschnittliche Kettenlänge der Peptide von weniger als 15 Aminosäuren besitzt,
   - und wobei mindestens 90 Gew.-% des Proteinhydrolysats ein Molekulargewicht von unter 1.000 D, bestimmt durch Gelpermeationschromatographie, besitzen.

2. Verfahren nach Anspruch 1, wobei die durchschnittliche Peptidkettenlänge in dem Proteinhydrolysat weniger als 10 Aminosäurereste beträgt.

3. Verfahren nach den Ansprüchen 1 bis 2, wobei das Proteinhydrolysat ein freies Aminosäurelevel von weniger als 4 Gew.-% des gesamten proteinhaltigen Materials besitzt.

4. Verfahren nach den Ansprüchen 1 bis 3, wobei das Proteinhydrolysat 20 Gew.-% oder mehr an Glutaminresten enthält.

5. Verfahren nach den Ansprüchen 1 bis 4, wobei das Proteinmaterial ein Getreideprotein ist.

6. Verfahren nach Anspruch 5, wobei das Proteinmaterial ein Weizengluten oder eine Unterfraktion dessen ist.

7. Kulturmedium für die in-vitro-Erhaltung oder das -Wachstum von eukaryontischen Zellen, das ein Glutamin-enthaltendes Proteinhydrolysat umfaßt, welches durch enzymatische Hydrolyse eines Proteinmaterials erhältlich ist, wobei das Proteinhydrolysat:

   - ein freies Aminosäurelevel von weniger als 15 Gew.-% des gesamten proteinhaltigen Materials,
   - eine durchschnittliche Kettenlänge der Peptide von weniger als 15 Aminosäuren besitzt,
   - und wobei mindestens 90 Gew.-% des Proteinhydrolysats ein Molekulargewicht von unter 1.000 D, bestimmt durch Gelpermeationschromatographie, besitzen.

8. Kulturmedium nach Anspruch 7, wobei die durchschnittliche Peptidkettenlänge in dem Proteinhydrolysat weniger als 10 Aminosäurereste beträgt.

9. Kulturmedium nach den Ansprüchen 7 bis 8, wobei das Proteinhydrolysat ein freies Aminosäurelevel von weniger als 4 Gew.-% des gesamten proteinhaltigen Materials besitzt.

10. Kulturmedium nach den Ansprüchen 7 bis 9, wobei das Proteinhydrolysat 20 Gew.-% oder mehr an Glutaminresten enthält.

11. Kulturmedium nach den Ansprüchen 7 bis 10, wobei das Proteinmaterial ein Getreideprotein ist.

12. Kulturmedium nach Anspruch 11, wobei das Proteinmaterial ein Weizengluten oder eine Unterfraktion dessen ist.

**Revendications**

1. Procédé pour l'entretien ou la croissance *in vitro* de cellules eucaryotes par utilisation d'un milieu de culture comprenant un hydrolysat de protéine contenant de la glutamine, obtenu par hydrolyse enzymatique d'une matière protéique, dans lequel l'hydrolysat de protéine présente :

- un taux d'acides aminés libres inférieur à 15% en poids de la matière protéique totale,
- une longueur de chaîne moyenne des peptides de moins de 15 acides aminés,
- et dans lequel au moins 90% en poids de l'hydrolysat de protéine ont un poids moléculaire inférieur à 1 000 D tel qu'il est déterminé par une chromatographie de perméation sur gel.

**2.** Procédé suivant la revendication 1, dans lequel la longueur de chaîne peptidique moyenne dans l'hydrolysat de protéine est inférieure à 10 résidus d'acide aminé.

**3.** Procédé suivant les revendications 1 et 2, dans lequel l'hydrolysat de protéine présente un taux d'acides aminés libres inférieur à 4% en poids de la matière protéique totale.

**4.** Procédé suivant les revendications 1 à 3, dans lequel l'hydrolysat de protéine contient 20% en poids ou plus de résidus glutamine.

**5.** Procédé suivant les revendications 1 à 4, dans lequel la matière protéique est une protéine de céréale.

**6.** Procédé suivant la revendication 5, dans lequel la matière protéique est un gluten de blé ou une fraction de celui-ci.

**7.** Milieu de culture pour l'entretien ou la croissance *in vitro* de cellules eucaryotes qui comprend un hydrolysat de protéine contenant de la glutamine, obtenu par hydrolyse enzymatique d'une matière protéique, dans lequel l'hydrolysat de protéine présente :

- un taux d'acides aminés libres inférieur à 15% en poids de la matière protéique totale,
- une longueur de chaîne moyenne des peptides de moins de 15 acides aminés,
- et dans lequel au moins 90% en poids de l'hydrolysat de protéine ont un poids moléculaire inférieur à 1 000 D tel qu'il est déterminé par une chromatographie de perméation sur gel.

**8.** Milieu de culture suivant la revendication 7, dans lequel la longueur de chaîne peptidique moyenne dans l'hydrolysat de protéine est inférieure à 10 résidus d'acide aminé.

**9.** Milieu de culture suivant les revendications 7 et 8, dans lequel l'hydrolysat de protéine présente un taux d'acides aminés libres inférieur à 4% en poids de la matière protéique totale.

**10.** Milieu de culture suivant les revendications 7 à 9, dans lequel l'hydrolysat de protéine contient 20% en poids ou plus de résidus glutamine.

**11.** Milieu de culture suivant les revendications 7 à 10, dans lequel la matière protéique est une protéine de céréale.

**12.** Milieu de culture suivant la revendication 11, dans lequel la matière protéique est un gluten de blé ou une fraction de celui-ci.